# EUROPÄISCHE PATENTSCHRIFT

(11) **EP 0 850 065 B1**
(45) Veröffentlichungstag und Bekanntmachung des Hinweises auf die Patenterteilung: **15.09.1999**
(21) Anmeldenummer: 96930118.3
(22) Anmeldetag: 29.08.1996
(51) Int. Cl.: A61K 31/695, A61K 9/70

(54) **TRANSDERMALES THERAPEUTISCHES SYSTEM FÜR SILATRANVERBINDUNGEN**
TRANSDERMAL THERAPEUTICAL SYSTEM FOR SILATRAN COMPOUNDS
SYSTEME THERAPEUTIQUE TRANSDERMIQUE POUR L'ADMINISTRATION DE COMPOSES DE SILATRAN

(30) Priorität: 15.09.1995 DE 19534201
(43) Veröffentlichungstag der Anmeldung: 01.07.1998
(73) Patentinhaber: LTS LOHMANN Therapie-Systeme GmbH, 56567 Neuwied (DE)
(72) Erfinder: HERRMANN, Fritz, D-56567 Neuwied (DE)
(74) Vertreter: Flaccus, Rolf-Dieter, Dr.
(86) Internationale Anmeldenummer: EP9603783
(87) Internationale Veröffentlichungsnummer: WO9709990

(56) Entgegenhaltungen:
- DE-A- 2 530 255
- FR-A- 2 321 295
- GB-A- 1 465 455
- US-A- 4 055 637
- DATABASE WPI Week 8622 Derwent Publications Ltd., London, GB; AN 86-140813 [22] XP002020455 A, & JP,A,61 076 494 (TOKUYAMA SODA KK) 18.April 1986

## Beschreibung

Die Erfindung betrifft ein transdermales therapeutisches System (TTS) zur Prophylaxe und/oder Behandlung von Woll- und Haarausfall.

Die Wirksamkeit von substituierten Silatranen als Woll- und Haarwuchsmittel ist aus der Deutschen Patentschrift 26 15 654 bekannt. Dabei wird besonders die gute Verträglichkeit der eingesetzten Verbindung hervorgehoben. Die Applikation erfolgt nach diesem Dokument entweder als Zusatz zur Nahrung, was besonders bei Tieren angezeigt ist, oder durch Einreiben mit Salben oder Suspensionen. Bei mehrmaligen Anwendungen pro Woche liegen die Behandlungszeiten im Bereich von Monaten. Da hierbei an die Compliance des Patienten erhebliche Ansprüche gestellt werden und außerdem die verabreichte Menge den Wirkstoffs wegen der bekannten Mängel einen Salben- oder Suspensionsauftrages schwer zu kontrollieren ist, kann der Stand der Technik nicht befriedigen.

Ausgehend hiervon ist es Aufgabe der Erfindung, eine neue Applikationsform anzugeben, durch welche die genannten Nachteile bei der Verwendung von substituierten Silatranen zur Prophylaxe und/oder Behandlung von Woll- und Haarausfall überwunden werden.

Diese Aufgabe wird dadurch gelöst, daß die substituierte Silatrane gemäß der Formel 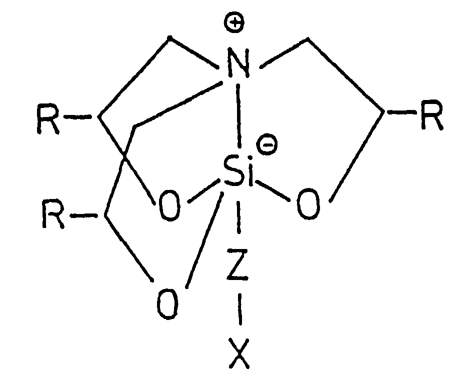 enthaltende Zubereitung in Form eines transdermalen therapeutischen Systems ausgebildet ist.

Hierbei bedeuten:
R = H oder CH₃,
Z = eine gegebenenfalls durch Chlor substituierte, 1 bis 3 C-Atome enthaltende zweibindige Gruppe
   und
X = H, niederes Alkyl, Aryl, Halogen, CF₃, CN, NH₂, SH, CNS, R₁M, (R₂O)₂P(O), R₃C(O)M mit
R₁ = Alkyl,geradkettig oder verzweigt mit 1 bis 4 C-Atomen, Aryl, Aralkyl, Alkaryl, M = O, S
R₂ = Methyl, Ethyl, R₃ = Alkyl mit 1 bis 4 C-Atomen, Fluoralkyl mit 1 bis 4 C-Atomen oder Aryl.

Therapeutische Systeme (TTS) enthalten einen oder mehrere Wirkstoffe, die in vorausbestimmter Rate kontinuierlich über einen festgesetzten Zeitraum an einen festgelegten Anwendungsort abgegeben werden (Heilmann, Therapeutische Systeme - Konzept und Realisation programmierter Arzneiverabreichung, 4. Auflage, Ferdinand Enke Verlag Stuttgart, 1984, S. 26).

Innerhalb dieser therapeutischen Systeme erhalten erfindungsgemäß transdermal wirkende Pflaster unter anderem auch deshalb den Vorzug, weil sich Silatrane aufgrund ihrer kugeligen Molekülgestalt zur Hautpenetration eignen. TTS in Pflasterform sind dem Fachmann bekannt. Sie umfassen im allgemeinen eine undurchlänsige Rückschicht, ein damit verbundenes Wirkstoffreservoir, bei Abwesenheit anderer Steuermechanismen eine die Abgabe den Wirkstoffs steuernde Membran, eine Haftklebeeinrichtung zur Befestigung des Pflasters auf der Haut und im Bedarfsfall eine vor Applikation des Pflasters ablösbare Schutzschicht. Wenn das Wirkstoffreservoir selbst haftklebend ist, kann die Herstellung des TTS durch homogenes Vermischen von Wirkstoff, Hilfsmittel und haftklebenden Polymeren in einem geeigneten Lösemittel, Aufstreichen der Lösung auf die Rückschicht, Entfernen des Lösemittels und Aufbringen einer Schutzschicht erfolgen. Auch die Umkehrung der Schichtenfolge ist möglich.

Grundsätzlich läßt sich das Reservoir auch aus der Schmelze herstellen.

Eine Synthese der erfindungngemäß einzusetzenden Wirkstoffe ist in der Deutschen Patentschrift 25 22 982 beschrieben. Wegen ihrer guten Verträglichkeit sind das 1-(Chlormethyl)silatran und das 1-(Ethoxy)-silatran besonders bevorzugt.

Die Konzentration mindestens einen Wirkstoffes aus der Silatrangruppe im Reservoir liegt bei 0,1 bis 50 Gew.-%, vorzugsweise bei 3 bis 25 Gew.-% und besonders bevorzugt bei 5 bis 15 Gew.-%. Die Dosis pro Pflaster läßt sich dann durch Wahl der Schichtdicke und flächenmäßige Ausdehnung des Reservoirs einstellen und richtet sich unter anderem nach dem Alter und der Hautoberfläche des Patienten, ferner nach der Art und dem Grad des Haarausfalls.

Zur Anwendung bei Tieren gelten vergleichbare Überlegungen.

Mit dem silatranhaltigen TTS wird eine Applikationsform für ein über längere Zeit anwendbares Mittel zur Prophylaxe und Behandlung des Woll- und Haarausfalls zur Verfügung gestellt, das eine Wirkungsdauer einer einzigen Applikation über Wochen und Monate ermöglicht und dabei eine gewünschte Dosierung des Wirkstoffs sicherstellt. Die systemische Wirkung der Silatrane macht darüber hinaus eine topische Applikation nicht zwingend.

## Patentansprüche

1. Zubereitung zur Prophylaxe und/oder Behandlung von Woll- oder oder Haarausfall, enthaltend den Wirkstoff Silatran nach der Formel 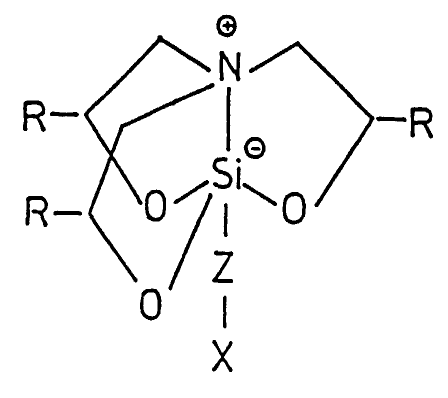 in der
R = H oder CH₃,
Z = eine gegenbenenfalls durch Chlor substituierte, 1 bis 3 C-Atome enthaltende zweibindige Gruppe
und
X = H, niederen Alkyl, Aryl, Halogen, CF₃, CN, NH₂, SH, CNS, R₁M, (R₂O)₂P(O), R₃C(O)M mit R₁ = Alkyl, geradkettig oder verzweigt mit 1 bis 4 C-Atomen, Aryl, Aralkyl, Alkaryl,
M = O, S, R₂ = Methyl, Ethyl, R₃ = Alkyl mit 1 bis 4 C-Atomen, Fluoralkyl mit 1 bis 4 C-Atomen oder Aryl bedeuten,
dadurch gekennzeichnet,
daß diese in Form eines transdermalen therapeutischen Systems vorliegt.

2. Zubereitung nach Anspruch 1, dadurch gekennzeichnet, daß sie als Pflaster vorliegt.

3. Zubereitung nach Anspruch 2, dadurch gekennzeichnet, daß das Pflaster eine undurchlässige Rückschicht, ein damit verbundenen Wirktoffreservoir, bei Abwesenheit anderer Steuermechanismen eine die Abgabe des Wirkstoffs steuernde Membran, eine Haftklebeeinrichtung zur Befestigung des Pflasters auf der Haut und gegebenenfalls eine vor der Applikation des Pflasters ablösbare Schutzschicht umfasst.

4. Zubereitung nach einem oder mehreren der Ansprüche 1 bis 3, dadurch gekennzeichnet, daß diese 0,1 bis 50 Gew.-%, vorzugsweise 3 bis 25 Gew.-% und besonders bevorzugt 5 bis 15 Gew.-% Wirkstoff enthält.

5. Zubereitung nach einem oder mehreren der Ansprüche 1 bis 4, dadurch gekennzeichnet, daß diese als Wirkstoff 1-(Chlormethyl)-silatran enthält.

6. Zubereitung nach einem oder mehreren der Ansprüche 1 bis 4, dadurch gekennzeichnet, daß diese als Wirkstoff 1-(Ethoxy)-silatran enthält.

## Claims

1. A preparation for the prophylaxis and/or treatment of wool or hair loss comprising the active substance silatrane of the formula 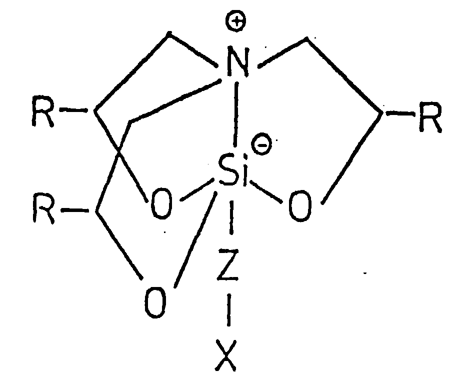 wherein
R = H or CH₃,
Z = a double-bond group comprising 1 to 3 C-atoms and optionally substituted by chlorine,
and
X = H, low alkyl, aryl, halogen, CF₃, CN, NH₂, SH, CNS, R₁M, (R₂O)₂P(O), R₃C(O)M with R₁ = alkyl, straight-chain or branched, with 1 to 4 C-atoms, aryl, aralkyl, alkaryl,
M = O, S, R₂ = methyl, ethyl, R₃ = alkyl with 1 to 4 C-atoms, fluoroalkyl with 1 to 4 C-atoms, or aryl,
characterized in that
it is present in the form a transdermal therapeutic system.

2. The preparation according to claim 1 characterized in that it is present as a patch.

3. The preparation according to claim 2 characterized in that the patch comprises an impermeable backing layer, an active substance reservoir connected therewith, a membrane controlling the release of the active substance in the absence of other control mechanisms, a pressure-sensitive adhesive device to fix the patch to the skin, and, if necessary, a protective layer removable prior to application.

4. The preparation according to one or several of claims 1 to 3 characterized in that it comprises 0.1 to 50%-wt., preferably 3 to 25%-wt., and most preferably 5 to 15%-wt. of active substance,

5. The preparation according to one or several of claims 1 to 4 characterized in that it comprises 1-(chloromethyl)-silatrane as active substance.

6. The preparation according to one or several of claims 1 to 4 characterized in that it comprises 1 -(ethoxy)-silatrane as active substance.

## Revendications

1. Formulation pour la prophylaxie et/ou le traitement de la chute de la laine ou des poils/cheveux, contenant le principe actif silatran répondant à la formule 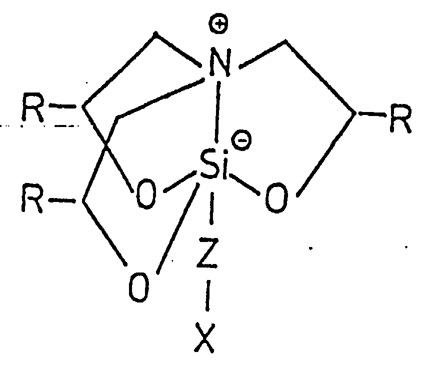 dans laquelle
R représente un atome d'hydrogène ou un groupe CH₃,
Z représente un groupe à liaison double contenant de 1 à 3 atomes de carbone, le cas échéant substitué par un ou plusieurs atomes de chlore,
et
X représente un atome d'hydrogène, un groupe alkyle inférieur, un groupe aryle, un atome d'halogène, un groupe CF₃, un groupe CN, un groupe NH₂, un groupe SH, un groupe CNS, un groupe R₁M, un groupe (R₂O)₂P(O), un groupe R₃C(O)M où R₁ représente un groupe alkyle à chaîne droite ou ramifiée contenant de 1 à 4 atomes de carbone, un groupe aryle, un groupe aralkyle, un groupe alkaryle,
M représente un atome d'oxygène, un atome de soufre, R₂ représente un groupe méthyle, un groupe éthyle, R₃ représente un groupe alkyle contenant de 1 à 4 atomes de carbone, un groupe fluoralkyle contenant de 1 à 4 atomes de carbone ou encore un groupe aryle,
caractérisée en ce que
cette formulation est présente sous la forme d'un système thérapeutique transdermique.

2. Formulation selon la revendication 1, caractérisée en ce qu'elle est présente sous la forme d'un emplâtre.

3. Formulation selon la revendication 2, caractérisée en ce que l'emplâtre comprend une couche dorsale imperméable, un réservoir de principe actif lié à cette dernière, en l'absence d'autres mécanismes de commande, une membrane commandant la libération du principe actif, un mécanisme autoadhésif pour la fixation de l'emplâtre sur la peau et le cas échéant, une couche de protection pelliculable avant l'application de l'emplâtre.

4. Formulation selon une ou plusieurs des revendications 1 à 3, caractérisée en ce qu'elle contient un principe actif à concurrence de 0,1 à 50% en poids, de préférence de 3 à 25% en poids, de manière particulièrement préférée de 5 à 15% en poids.

5. Formulation selon une ou plusieurs des revendications 1 à 4, caractérisée en ce qu'elle contient, à titre de principe actif, du 1-(chlorométhyl)-silatran.

6. Formulation selon une ou plusieurs des revendications 1 à 4, caractérisée en ce qu'elle contient, à titre de principe actif, du 1-(éthoxy)-silatran.
